# EUROPÄISCHE PATENTSCHRIFT

(11) **EP 1 165 016 B1**
(45) Veröffentlichungstag und Bekanntmachung des Hinweises auf die Patenterteilung: **25.05.2005**
(21) Anmeldenummer: 00926784.0
(22) Anmeldetag: 31.03.2000
(51) Int. Cl.: A61K 6/10, C08L 83/04, C08L 71/02

(54) **ABFORMMASSEN AUF SILIKONBASIS MIT VERBESSERTEM STANDVERMÖGEN**
SILICON-BASED IMPRESSION COMPOUNDS WITH IMPROVED NON-SAG PROPERTIES
MATIERE MOULABLE A BASE DE SILICONE A RESISTANCE AU FLUAGE AMELIOREE

(30) Priorität: 01.04.1999 DE 19915004
(43) Veröffentlichungstag der Anmeldung: 02.01.2002
(73) Patentinhaber: 3M ESPE AG, 82229 Seefeld (DE)
(72) Erfinder: ZECH, Joachim, D-82229 Seefeld (DE); WANEK, Erich, D-86916 Kaufering (DE)
(86) Internationale Anmeldenummer: PCT/EP2000/002854
(87) Internationale Veröffentlichungsnummer: WO 2000/059453

(56) Entgegenhaltungen:
- EP-A- 0 287 092
- WO-A-87/03001
- WO-A-96/26246
- W. GERHARTZ ET AL.: "Ullman's Encyclopedia of Industrial Chemistry, Vol. A 8" 1987 , VCH VERLAG , WEINHEIM, DE XP002143931 Seite 289, linke Spalte

## Beschreibung

Die Erfindung betrifft Abformmassen auf Silikonbasis mit verbessertem Standvermögen. Insbesondere betrifft die Erfindung dentale A-Silikon-Abformmassen, deren Standvermögen durch Zugabe geringer Mengen von Polyalkylenoxiden und/oder deren Derivaten mit einer mittleren Molmasse M_{w} > 3000 erhöht wurde.

In der Zahnmedizin wird anhand von Modellen Zahnersatz angefertigt..Hierzu wird zunächst mittels einer Abformmasse ein räumliches Negativ der Kiefersituation hergestellt, wobei beispielsweise die plastische, noch nicht abgebundene Abformmasse auf einem Abdrucklöffel in den Mund des Patienten eingeführt wird und dort erstarrt. Es ist auch möglich, die besonders genau abzuformenden Stellen des Kiefers zunächst mit Abformmasse aus einer sogenannten Elastomerspritze zu umspritzen und danach erst einen mit Abformmasse befüllten Löffel in den Mund des Patienten zur endgültigen Abdrucknahme zu bringen. Die Abformmasse erstarrt letztlich zu einer harten oder elastischen Masse, die nach dem Herausnehmen die eingangs erwähnte negative Form darstellt.

Als elastische Abformmaterialien werden die verschiedensten Materialklassen eingesetzt, beispielsweise Polyether und Silikone, die durch eine chemische Reaktion zum Elastomer vernetzt werden. Bei den Silikonen sind kondensations- und additionsvernetzende Systeme erhältlich. Die kondensationsvernetzenden Silikone (C-Silikone) vernetzen in der Regel durch titan- oder zinnkatalysierte Reaktion des hydroxyterminierten Polysiloxans mit Siliciumalkoxyverbindungen unter Abspaltung eines Alkohols (Kondensation). Bei den additionsvernetzenden Silikonen (A-Silikone) erfolgt die Vernetzung in der Regel durch platinkatalysierte Reaktion von ungesättigten Kohlenwasserstoffendgruppen des Polysiloxans mit Si-H-Gruppen eines Hydrogenpolysiloxans (Hydrosilylierung, Addition). A-Silikon-Abformmassen sind beispielsweise aus der EP-A-0 162 211, DE-A-40 31 759 und EP-A-0 166 107 sowie aus der Fachliteratur, beispielsweise aus R.G. Graig, Restaurative Dental Materials, The C.V. Moosbe-Comp., St. Louis, 1980, S. 195ff bekannt.

Die Silikonmassen werden üblicherweise als Zweikomponentensystem - in Form getrennt verpackter Basis- und Katalysatorpasten - geliefert, wobei die Komponenten vor der Anwendung automatisch oder manuell in einem bestimmten Mischungsverhältnis dosiert, gemischt und anschließend in den Mund des Patienten gebracht werden. Dort härten sie dann innerhalb einer vorgegebenen Zeitspanne von wenigen Minuten aus.

Allgemein bestehen die Basispasten meist aus vinyl-terminiertem Silikonöl, Füller und Quervemetzer und die Katalysatorpasten aus vinyl-terminiertem Silikonöl, Füller und Katalysator.

Ein Nachteil vieler bekannter Silikonmassen besteht darin, daß sie im nichtausgehärteten Zustand eine relativ geringe Standfestigkeit aufweisen. Diesen Nachteil versucht man durch verschiedene Möglichkeiten zu umgehen:
◆ Hoher Befüllungsgrad der Pasten mit groben anorganischen Füllstoffen
◆ Zusatz von pyrogenen Kieselsäuren oder von Diatomeenerde (WO-96/32088)
◆ Zusatz von Wachsen (DE-A-195 17 962)

Nachteilig bei diesen Lösungen ist jedoch, daß einerseits schlechte Fließfähigkeit oder mangelhafte Zeichnungsschärfe, verursacht durch die Füller selbst, festgestellt wird und andererseits die Zusätze zum Ausbluten neigen, da sie in nicht unerheblichen Mengen, im Falle der DE-A-195 17 962 von bis zu 40 Gew.-% zugesetzt werden. Auch ist ein Abnehmen der Shore-Härte und eine Verschlechterung der Beschneidbarkeit sowie der Lagerstabilität beobachtet worden.

Von praxisrelevantem Nachteil ist ferner, daß Pasten mit hohem Füllstoffanteil oder viskosen Zusätzen, wie Wachsen, nur schwer aus ihren Behältnissen ausdrückbar sind. Dem Zahnarzt oder seiner Helferin ist es unter Umständen mit einer Hand nicht mehr möglich, die Pasten auszubringen. Elektrisch betriebene Mischgeräte können Schaden nehmen, wenn die Ausdruckkräfte zu sehr ansteigen.

Die WO 87/03001 beschreibt additionsvernetzende Zweikomponenten-Silikonmassen, die Polyalkylenoxid (bzw. dessen Derivate) auf der Basis von Ethylen- und/oder Propylenoxid mit Molmassen im Bereich zwischen 1800 und 3000 enthalten und als Zahnabdruckmassen Verwendung finden können.

Die WO 96/26246 betrifft standfeste Abformmassen auf der Basis von additionsvernetzenden Silikonzusammensetzungen und natürlichen und synthetischen Wachsen in Mengen von vorzugsweise 5 bis 10 Gew.-%, deren Molekulargewicht vorzugsweise unterhalb 2000 liegen soll.

Ullmann's Encyclopedia of Indusstrial Chemistry, VCH Verlag, Weinheim, Vol. A 8, 1987, Seite 289 beschreibt unter dem Stichwort "Dental Waxes" natürliche und synthetische Wachse als Materialien, die in der Dentaltechnik verwendet werden können. Molekulargewichte sind nicht angegeben.

Die EP-A-0 287 092 beschreibt ganz allgemein, daß Ethylenoxidcopolymere in Kunststoffen als Additiv eingesetzt werden können, ohne daß es zu Ausblühungen kommt.

Eine möglichst hohe Standfestigkeit wird beispielsweise bei Bißregistraten benötigt. Auch bei Abformmassen für alle anderen Indikationen ist eine hohe Standfestigkeit wünschenswert, damit die nicht-abgebundene Masse beim Einbringen in den Mund des Patienten nicht aus dem Abformlöffel in den Rachen des Patienten fließt und damit Würgereiz hervorruft oder damit beim Umspritzen einzelner Kieferpartien die Massen nicht schwerkraftbedingt im Falle des Oberkiefers abtropfen oder im Falle des Unterkiefers abfließen und die präparierte Stelle im Kiefer, wo besondere Präzision notwendig ist, freisetzen. Es besteht daher die Notwendigkeit, Möglichkeiten zu finden, die Standfestigkeit von Abformmassen zu erhöhen, ohne daß die vorher beschriebenen Nachteile der hohen Ausdruckkräfte und des Ausblutens beobachtet werden.

Überraschenderweise wurde gefunden, daß Zusätze von 0,001 bis 1,0 Gew.-% von Polyalkylenoxiden und/oder deren Derivaten mit einer mittleren Molmasse M_{w} > 3000 zu A-Silikon-Abformmassen die Standfestigkeit dieser Massen dauerhaft erhöhen, ohne daß die Ausdrückbarkeit und die Shore-Härte beeinträchtigt werden.

Das erfindungsgemäße Abformmaterial enthält die folgenden Bestandteile:
(a) Organopolysiloxane mit mindestens zwei ungesättigten Gruppen im Molekül,
(b) Organohydrogenpolysiloxane mit mindestens drei Si-H-Gruppen im Molekül,
(c) gegebenenfalls Organopolysiloxane ohne reaktive Gruppen,
(d) Platinkatalysator,
(e) gegebenenfalls Hydrophilierungsmittel,
(f) Füllstoff,
(g) gegebenenfalls weitere übliche Zusatz-, Hilfs- und Farbstoffe,
(h) Polyalkylenoxide und/oder deren Derivate mit einer mittleren Molmasse M_{w} > 3000 zu 0,001 bis 1,0 Gew.-%, bezogen auf die Gesamtmasse des ausgehärteten Materials.

In den Zeichnungen zeigen:
- die Abbildung 1:: An der Spitze abgesägte Elastomerspritze, die mit angemischter Abformmasse (A) befüllt ist,
- die Abbildung 2:: Ober den Rand der Glasplatte (G), in der die Spritze montiert ist, hinausragende Abformmasse (A) in Richtung (X) parallel zur Spritze.
- die Abbildung 3:: Darstellung des Winkels (φ) = 90° zwischen einer Parallelen (Y) der Glasplatte (G) und einer idealisierten Geraden (X) durch den Strang der ausgebrachten Abformmasse (A)
- die Abbildung 4:: Darstellung des Winkels (φ) = 0° zwischen einer Parallelen (Y) der Glasplatte (G) und einer idealisierten Geraden (X) durch den Strang der ausgebrachten Abformmasse (A)

Die prinzipielle Verwendung von Zusätzen zu Kieselsäure-haltigen Silikonöl-Systemen, um deren Standfestigkeit zu beeinflussen, ist allgemein bekannt. In der Broschüre "Additives for CAB-O-SIL®", CABOT GmbH, Hanau ist eine Auflistung der verschiedensten Additivklassen für die diversen Zielsetzungen gegeben. Die erfindungsgemäße Verwendung von polymeren Polyalkylenoxiden und/oder deren Derivaten mit einer mittleren Molmasse M_{w} > 3000 zur Lösung der hier gestellen Aufgabe wird darin jedoch nicht beschrieben, noch wird sie durch diese Broschüre nahegelegt.

Bevorzugt sind als Komponente (a) Diorganopolysiloxane mit terminalen Triorganosiloxygruppen, von denen mindestens eine der drei organischen Gruppen eine Vinylgruppe ist. Bevorzugte Diorganosiloxane dieser Struktur werden durch folgende Formel (1) wiedergegeben: 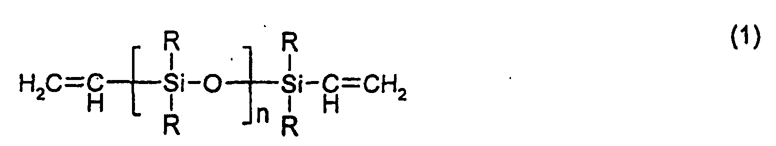 in der R eine unsubstituierte oder substituierte monovalente Kohlenwasserstoffgruppe repräsentiert, die bevorzugt frei von aliphatischen Mehrfachbindungen ist und n eine ganze Zahl darstellt. Mindestens 50 % der Reste R sind bevorzugt Methylgruppen. Beispiele für andere Gruppen R sind Ethyl-, Vinyl- und 3,3,3-Trifluorpropylgruppen. Der Wert von n soll so liegen, daß das Polymer bei 25°C eine Viskosität zwischen 200 bis 200.000 mPa.s, bevorzugt 1000 bis 10.000 mPa.s aufweist. Derartige Moleküle sind in der US-A-4 035 453 beschrieben, deren Offenbarung hier soweit mit umfaßt sein soll.

Die Herstellung der Komponenten (a) erfolgt nach üblichen Verfahren, die z.B. in W. Noll, "Chemie und Technologie der Silikone", Verlag Chemie Weinheim, 2. Auflage 1964, Seite 162-206 oder J. Burghardt, Chemie und Technologie der Polysiloxane in "Silikone, Chemie und Technologie", Vulkan Verlag, Essen, 1989, Seiten 23-37 dargestellt sind.

Besonders bevorzugt sind lineare Polydimethylsiloxane obiger Struktur mit den angegebenen Viskositätsbereichen, bei denen die Endgruppen aus Dimethylvinylsiloxyeinheiten bestehen und die weiteren Substituenten R in der Kette aus Methylgruppen bestehen.

Komponente (b) ist bevorzugt ein Organopolysiloxan mit mindestens drei Sigebundenen Wasserstoffatomen pro Molekül. Dieses Organopolysiloxan enthält bevorzugt von 0,01 bis 1,7 Gew.-% siliciumgebundenen Wasserstoff. Die Silikonvalenzen, die nicht mit Wasserstoff- oder Sauerstoffatomen abgesättigt sind, werden mit monovalenten Kohlenwasserstoffradikalen abgesättigt, die frei von aliphatischen Mehrfachbindungen sind. Die Kohlenwasserstoffradikale können substituiert oder unsubstituiert sein. Mindestens 50%, bevorzugt 100%, der Kohlenwasserstoffradikale, die an Siliciumatome gebunden sind, bestehen aus Methylradikalen. Auch derartige Komponenten sind in den oben genannten Literaturstellen hinsichtlich Struktur und Herstellung beschrieben.

Die Mengenverhältnisse der Komponenten (a) und (b) sind bevorzugt derart gewählt, daß pro Mol an ungesättigter Doppelbindung der Komponente (a) 0,75 bis 5 Mol Si-H-Einheiten aus der Komponente (b) vorliegen. Die Summe der Komponenten (a) und der Komponente (b) liegt im Bereich von 10 bis 40 Gew.-%, bezogen auf das Gesamtgewicht aller Komponenten. Bevorzugt liegen sie im Bereich von 15 bis 30 Gew.-%.

Geeignete Komponenten (c) sind polymere Organosiloxane ohne reaktive Substituenten, wie sie z.B. in W. Noll "Chemie und Technologie der Silikone", Verlag Chemie Weinheim, 1968, Seite 212 ff. beschrieben sind. Es handelt sich bevorzugt um lineare, verzweigte oder cyclische Organopolysiloxane, bei denen sämtliche Siliciumatome von Sauerstoffatomen oder monovalenten Kohlenwasserstoffradikalen umgeben sind, wobei die Kohlenwasserstoffradikale substituiert oder unsubstituiert sein können. Die Kohlenwasserstoffradikale können Methyl, Ethyl, C₂-C₁₀-Aliphaten, Trifluorpropylgruppen, sowie aromatische C₆-C₁₂-Substituenten sein. Die Komponente (c) trägt zur Verdünnung und Aufweitung des Netzwerkes bei und wirkt als Weichmacher für das ausgehärtete Material. Als relativ günstige Komponente trägt sie auch bei zur Reduktion der Herstellungskosten der erfindungsgemäßen Abformmassen.

Besonders bevorzugt als Komponente (c) sind Polydimethylsiloxane, welche Trimethylsiloxy-Endgruppen aufweisen. Die Viskosität der Komponente (c) liegt bevorzugt im Bereich von 10 bis 20.000 mPa.s, besonders bevorzugt 10 bis 1.000 mPa.s. Die Menge an Komponente (c) beträgt 0 bis 40 Gew.-%, bevorzugt 5 bis 40 Gew.-%, besonders bevorzugt 15 bis 30 Gew.-%.

Komponente (d) ist bevorzugt ein Platinkomplex, der aus Hexachlorplatinsäure durch Reduktion mit Tetramethyldivinyldisiloxan hergestellt wurde. Diese Verbindungen sind an sich bekannt. Geeignet sind auch andere Platinverbindungen, die die Additionsvernetzung beschleunigen. Gut geeignet sind z.B. Platin-Siloxan-Komplexe, wie sie beispielsweise in den US-A-3 715 334, US-A-3 775 352 und US-A-3 814 730 beschrieben sind. Der Platinkatalysator wird in Mengen von 0,00005 bis 0,05 Gew.-%, vorzugsweise 0,0002 bis 0,04 Gew.-%, jeweils berechnet als elementares Platin und bezogen auf das Gesamtgewicht der mit den Komponenten (a) bis (h) vorliegenden Masse, eingesetzt. Zur Steuerung der Reaktivität kann es notwendig sein, daß ein Inhibitor zugesetzt werden muß, der die vorzeitige Vernetzung zum Elastomeren behindert. Derartige Inhibitoren sind bekannt und z.B. in US-A-3 933 880 beschrieben.

Beispiele hierfür sind acetylenisch ungesättigte Alkohole, wie 3-Methyl-1-butin-3-ol, 1-Ethinylcyclohexan-1-ol, 3,5-Dimethyl-1-hexin-3-ol und 3-Methyl-1-pentin-3-ol. Beispiele für Inhibitoren auf Vinylsiloxanbasis sind 1,1,3,3-Tetramethyl-1,3-divinylsiloxan und vinylgruppenhaltige Poly-, Oligo- und Disiloxane.

Die nach der Vermischung ausgehärteten, erfindungsgemäßen Massen sind nicht hydrophil und weisen ohne den Zusatz von Hydrophilierungsmitteln einen Kontakt- bzw. Randwinkel auf, der vorzugsweise größer als 70°, besonders bevorzugt größer als 80°, ist. Gegebenenfalls wird deshalb den Massen Komponente (e) zugesetzt, welche ein eine hydrophile Natur verleihendes Mittel oder Hydrophilierungsmittel ist, das den Randwinkel eines Tropfens Wasser oder wäßriger Zusammensetzung (z.B. Gipssuspension, etc.) gegenüber der Silikonzusammensetzung erniedrigt und damit eine bessere Benetzbarkeit der Gesamtzusammensetzung im feuchten Mundmilieu und damit ein besseres Anfließverhalten der Pasten hervorruft. Die Hydrophilierungsmittel sind bevorzugt nicht mit reaktiven Gruppen versehen, so daß kein Einbau in das Polysiloxannetzwerk stattfindet. Geeignete Hydrophilierungsmittel sind bevorzugt nicht einbaubare Benetzungsmittel aus der Gruppe der hydrophilen Silikonöle, die in der WO-87/03001 und in der EP-B-0 231 420 beschrieben sind, auf deren Offenbarung hier insoweit Bezug genommen werden soll. Bevorzugt sind ferner die ethoxylierten Fettalkohole, die in der EP-B-0 480 238 beschrieben sind. Desweiteren sind bevorzugte Hydrophilierungsmittel die aus der WO-96/08230 bekannten Polyethercarbosilane. Bevorzugt sind auch die in der WO-87/03001 beschriebenen nicht-ionischen perfluoralkylierten oberflächenaktiven Substanzen. Ebenfalls bevorzugt sind die nicht-ionischen oberflächenaktiven Substanzen, die in der EP-B-0 268 347 beschrieben sind, d.h. die darin aufgeführten Nonylphenolethoxylate, Polyethylenglykol-mono- und -diester, Sorbitanester, sowie Polyethylenglykol-mono- und -diether. Die Einsatzmengen der Hydrophilierungsmittel betragen 0 bis 10 Gew.-%, bezogen auf das Gesamtgewicht aller Komponenten, bevorzugt 0 bis 2 Gew.-% und besonders bevorzugt 0 bis 1 Gew.-%.

Der Randwinkel wird nach der Methode des liegenden Tropfens gemäß DE-A-4433139 gemessen. Hierbei erfolgt die Bestimmung mit einem Kontaktwinkelmesssystem G1/G40 (Krüss). Dieses Messgerät ermöglicht die präzise Abbildung von Tropfenprofilen auf Festkörperoberflächen. Das Messsystem umfasst weiterhin einen Videotubus mit Strahlenteiler, so dass die gleichzeitige Beobachtung eines Tropfens durch das Goniometerokular (Tropfengröße) und die Videokamera (digitale Bildauswertung) ermöglicht wird. Die Messung erfolgt am liegenden Tropfen bei 23°C und 50% relativer Luftfeuchtigkeit. 30 Minuten nach Anmischbeginn der Massen wird ein stets gleich großer Tropfen einer auf 23°C gesättigten Calciumsulfatdihydratlösung auf das zwischen Glasplatten zu glatter Oberfläche ausgehärtete Elastomer aufgetragen und sofort mit der Messung begonnen. Der 10-Sekundenwert wird zur Auswertung herangezogen.

Komponente (h) ist ein Polyalkylenoxid und/oder -derivat mit einer mittleren Molmasse M_{w} > 3000. Hierbei sind entweder chemisch inerte oder reaktive Endgruppen enthaltende Moleküle erfindungsgemäß.

Zur Bestimmung der Molmasse der Verbindungen gemäß Komponente (h) können dem Fachmann bekannte Methoden in Abhängigkeit vom Vorhandensein reaktiver Endgruppen und der Molmasse verwendet werden, beispielsweise Endgruppenbestimmung, Messung des osmotischen Drucks, isothermische Destillation (Methode der isopiestischen Lösungen), Auszählung mittels Elektronenmikroskopie, Diffusionsmessung, Sedimentation, Ultrazentrifugierung, Fällungs- und Trübungstitration, Messung von Viskosität, Lichtstreuung, Dialysengeschwindigkeit. Geeignet bei der Bestimmung der Molmasse von reaktiven Endgruppen-, beispielsweise OH-Gruppen-haltigen Polyalkylenoxiden ist u.a. die Titration der Endgruppen nach DAB 10 als Bestimmung der Hydroxylzahl. Diese Methode ist im Rahmen der vorliegenden Erfindung für Polyalkylenoxide gemäß Komponente (h) angewandt worden. Für Polyalkylenoxidderivate gemäß Komponente (h) kann nach vorheriger Umwandlung der Endgruppen in titrierbare Gruppen die gleiche Methode angewandt werden.

Bevorzugte Vertreter der Komponente (h) sind Polyalkylenoxidderivate nach EP-B-0 421 371 (R≠H) sowie daraus abzuleitende Polyalkylenoxide (R=H). Beide Substanzen folgen der angegebenen allgemeinen Formel (2):

R-X-[(CH₂)ₙ-(CHR')-O]ₖ-[(CH₂)ₘ-(CHR")-O]ₗ-(CH₂)ₘ-(CHR")-X-R (2)

worin bedeuten:
- n =: 1 bis 6, vorzugsweise 1 bis 4, insbesondere 1, wobei n innerhalb der Kette variieren kann,
- m =: 1 bis 6, vorzugsweise 1 bis 4, insbesondere 3, wobei m innerhalb der Kette variieren kann,
- k, I =: 2 bis 500, bevorzugt 4 bis 250, insbesondere 10 bis 200,
- R', R" =: H, Methyl, Ethyl, bevorzugt ist R'= R" = H,
- X =: S, O NH, vorzugsweise O,
- R =: H oder C₁₋₁₈-, vorzugsweise C₁₋₁₂-, insbesondere C₁₋₆-Alkyl, besonders bevorzugt C₁₋₃-Alkyl, oder Carbonyl-C₁₋₁₇-, vorzugsweise Carbonyl-C₁₋₁₁-, insbesondere Carbonyl-C₁₋₅-Alkyl, besonders bevorzugt Carbonyl-C₁₋₃-Alkyl, oder ein Rest der allgemeinen Formel (3):

-CO-Xₚ-R''' (3)
- worin R''' =: C₁₋₁₈-, vorzugsweise C₁₋₁₂-, insbesondere C₁₋₆-Alkyl, besonders bevorzugt C₁₋₃-Alkyl, und/oder C₆₋₁₈-Aryl, vorzugsweise C₆₋₁₂-Aryl bedeutet und X die oben angegebene Bedeutung hat und p = 0 oder 1 ist, wobei p = 0 bevorzugt ist,
- und worin R: bevorzugt nicht H ist,
wobei die mit den Symbolen k und I indizierten Klammerausdrücke regelmäßig oder unregelmäßig alternierend oder in Blockform angeordnet sein können.

Ein besonders bevorzugter Vertreter hieraus ist das Diacetat der nachfolgenden Formel (4), welches zu den chemisch inerten Varianten gehört: 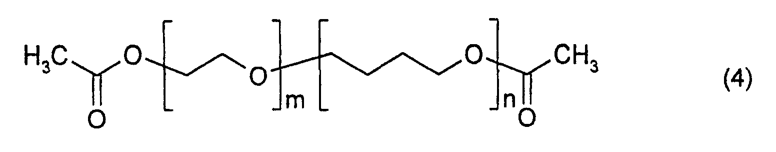 oder das Derivat der Formel (5) 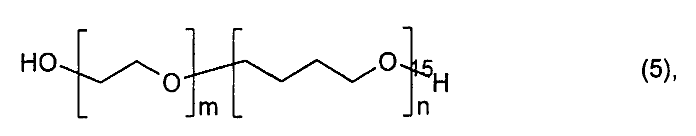 wobei das Verhältnis von m : n im Bereich von 1 : 3 bis 1 : 4 liegt. Die mittlere Molmasse M_{w} beträgt vorzugsweise ≈ 6000.

Bevorzugt sind weiterhin Blockcopolymere des Propylenoxids und Ethylenoxids, wie etwa Derivate des Propylenglykols und des Ethylendiamins, welche von der Firma Wyandotte unter den Bezeichnungen Pluronics und Tetronics vertrieben werden. Ebenso möglich sind ethoxylierte Polypropylenoxide unterschiedlicher Kettenlängen (Synperonic-Typen), SB 169 P (Polyether Fa. Goldschmidt) sowie Tegopren-7006.

Die erfindungsgemäßen Polyalkylenoxide weisen bevorzugt keinen tensidischen Charakter auf. Außerdem sind sie in bevorzugter Weise derivatisiert, so daß sie mit Bestandteilen der Matrix keine Reaktionen, insbesondere über OH- bzw. NH-Gruppen, eingehen können.

Innerhalb der erfindungsgemäßen Massen können selbstverständlich auch Gemische verschiedener Vertreter der Komponente (h) eingesetzt werden. Der Gesamtgehalt an Komponente (h) beträgt 0,001 bis 1,0 Gew.-%, vorzugsweise 0,01 bis 0,5 Gew.-% und besonders bevorzugt 0,02 bis 0,3 Gew.-%, jeweils bezogen auf das Gesamtgewicht der Masse.

Zu den einsetzbaren Füllstoffen gemäß Komponente (f) gehören nicht verstärkende Füllstoffe mit einer BET-Oberfläche von bis zu 50 m²/g, wie Quarz, Christobalit, Calciumsilikat, Zirkoniumsilikat, Montmorillonite wie Benthonite, Zheolite, einschließlich der Molekularsiebe, wie Natriumaluminiumsilikat, Metalloxidpulver, wie Aluminium- oder Zinkoxide bzw. deren Mischoxide, Bariumsulfat, Calciumcarbonat, Gips, Glas- und Kunststoffpulver. Zu möglichen Füllstoffen gehören auch verstärkende Füllstoffe mit einer BET-Oberfläche von mehr als 50 m²/g, wie z.B. pyrogene oder gefällte Kieselsäure und Siliciumaluminiummischoxide mit großer BET-Oberfläche. Die genannten Füllstoffe können hydrophobiert sein, beispielsweise durch die Behandlung mit Organosilanen bzw. -siloxanen oder durch die Veretherung von Hydroxylgruppen zu Alkoxygruppen. Es kann eine Art von Füllstoff, es kann auch ein Gemisch von mindestens zwei Füllstoffen eingesetzt werden. Die Kornverteilung wird vorzugsweise so gewählt, daß keine Füllstoffe mit Korngrößen > 50 µm enthalten sind. Der Gesamtgehalt der Füllstoffe (f) liegt im Bereich von 10 bis 79,99895 Gew.-%, bevorzugt 30 bis 60 Gew.-%, bezogen auf das Gesamtgewicht der Masse.

Besonders bevorzugt ist eine Kombination aus verstärkenden und nicht verstärkenden Füllstoffen. Hierbei liegen die verstärkenden Füllstoffe in Mengenbereichen von 1 bis 10 Gew.-%, insbesondere 2 bis 5 Gew.-%. Die Differenz zu den genannten Gesamtbereichen, also 9 bis 69,99895 Gew.-%, insbesondere 28 bis 55 Gew.-%, bilden die nicht verstärkenden Füllstoffe.

Bevorzugt als verstärkende Füllstoffe sind pyrogen hergestellte hochdisperse Kieselsäuren, die bevorzugt durch Oberflächenbehandlung hydrophobiert worden sind. Die Oberflächenbehandlung kann beispielsweise mit Dimethyldichlorsilan, Hexamethyldisilazan, Tetramethylcyclotetrasiloxan oder Polymethylsiloxanen erfolgen. Die Oberflächen geeigneter pyrogener Kieselsäuren betragen bevorzugt > 50 m²/g, insbesondere 80 bis 150 m²/g. Die Anwesenheit der oberflächenbehandelten pyrogenen Kieselsäuren trägt zur Einstellung der Konsistenz und zur Verbesserung der Standfestigkeit der Pasten bei. Bei Mengen von < 1 Gew.-% ist in der Regel kein merkbarer Einfluß auf die Standfestigkeit feststellbar, Mengen von > 10 Gew.-% führen in der Regel zu einer zu starken Verdickung der Pasten, so daß keine ausreichende Fließfähigkeit mehr erhalten werden kann.

Geeignete Produkte sind beispielsweise in den Prospekten der Fa. Degussa (Aerosil-Produkte, Schriftenreihe Pigmente, Nr. II, 5. Auflage, 1991, auf Seite 79) sowie der Fa. Cabot Corp. (Cabosil-Produkte, "CAB-O-SIL Fumed silica in Adhesives and Sealants, Cabot, 1990) beschrieben.

Besonders bevorzugte nichtverstärkende Füllstoffe sind Quarze, Christobalite und Natriumaluminiumsilikate, die oberflächenbehandelt sein können. Die Oberflächenbehandlung kann prinzipiell mit den gleichen Methoden erfolgen wie im Fall der verstärkenden Füllstoffe beschrieben worden ist.

Weiterhin können die erfindungsgemäßen Abformmassen als Komponente (g) gegebenenfalls Zusätze wie Weichmacher, Pigmente, Antioxidationsmittel, Trennmittel, u.a. enthalten. Ebenso können auch beispielsweise fein verteiltes Palladium oder Platin als Wasserstoffabsorber enthalten sein. Die Metalle können dabei auch auf Trägermaterialien aufgebracht sein. Die erfindungsgemäßen Massen enthalten derlei Zusatzstoffe in Mengen von vorzugsweise 0 bis 2 Gew.-%, besonders bevorzugt von 0,1 bis 1 Gew.-%.

Die Massen werden hergestellt durch Vermischen der Komponenten (a) bis (h) und härten in einer als Hydrosilylierung bezeichneten Additionsreaktion aus, bei der sich unter dem Einfluß des Platinkatalysators (d) die Si-H-Gruppen der Komponente (b) an die ungesättigten Gruppen der Komponente (a) addieren. Aus Lagerstabilitätsgründen ist es bevorzugt, die Massen in einer zweikomponentigen Darreichungsform zu formulieren, bei der die gesamte Komponente (b) in einer sogenannten Basispaste untergebracht ist. Räumlich getrennt hiervon wird die gesamte Komponente (d) in einer sogenannten Katalysatorpaste untergebracht. Die Komponente (a) kann entweder in der Katalysator- oder Basispaste untergebracht sein, wobei bevorzugt je ein Teil der Komponente (a) in der Basis- und ein Teil der Komponente (a) in der Katalysatorpaste untergebracht ist. Die Komponenten (c), (e), (f), (g) und (h) können in ihrer Gesamtmenge in der Katalysator- oder in der Basispaste untergebracht sein, wobei bevorzugt ist, daß jeweils ein Teil der jeweiligen Komponente in der Katalysator- und ein Teil in der Basispaste untergebracht ist. Besonders bevorzugt ist, daß die Komponenten (e) und (h) nur in der Basispaste enthalten sind.

Die Volumenverhältnisse von Katalysator- und Basispaste können 10:1 bis 1:10 betragen. Besonders bevorzugte Volumenverhältnisse von Basis- : Katalysatorpaste sind 1:1 und 5:1 (5 Teile Basis- : 1 Teil Katalysatorpaste).

Die Erfindung soll nachfolgend durch Beispiele näher erläutert werden, ohne daß sie durch diese limitiert werden soll.

### Herstellungsbeispiel Additiv

600 g eines Copolymers aus Tetrahydrofuran und Ethylenoxid (im Verhältnis 2 : 1, Molekulargewicht 6.000; im Rahmen dieser Anmeldung "Diol 6000" genannt) werden in 1,3 l Cyclohexan aufgelöst. Anschließend werden 19,4 g Acetanhydrid und 3,5, g Toluolsulfonsäure zugegeben, und es wird 4 Stunden unter Rückfluß erhitzt. Die Aufarbeitung erfolgt mittels zweimaliger Extraktion mit wäßriger Natronlauge (2n), einmaliger Extraktion mit wäßriger Schwefelsäure sowie zweimaliger Extraktion mit destilliertem Wasser. Anschließend wird mit Natriumsulfat getrocknet und das Lösungsmittel abdestilliert. Man erhält 500 g Polyalkylenoxidbisacetat.

### Vergleichsbeispiel

Basis- und Katalysatorpaste werden analog dem Anwendungsbeispiel hergestellt, jedoch wird kein Vertreter der erfindungsgemäßen Komponente (h) zugesetzt. Die Standfestigkeit wird analog dem folgenden Test durchgeführt.

### Anwendunasbeispiele

### Basispaste:

Es werden 24,9 g α-ω-terminiertes Polydimethylsiloxan mit einer Viskosität von 200 mPa.s bei 23°C, 3,2 g Poly(methyl)hydrogensiloxan mit einer Viskosität von 35 mPa.s bei 23°C, 2,0 g Silikonöl mit einer Viskosität von 50 mPa.s bei 23°C und 0,02 g Pigment zusammen mit 3,1 g silanisierter pyrogener Kieselsäure und 66,6 g Quarz in einem Kneter vermischt.

### Katalysatorpaste:

Es werden 27,0 g α-ω-terminiertes Polydimethylsiloxan mit einer Viskosität von 200 mPa.s bei 23°C und 2,0 g Silikonöl mit einer Viskosität von 50 mPa.s bei 23°C zusammen mit 2,9 g silanisierter pyrogener Kieselsäure, 68,0 g Quarz und 0,3 g Platinkatalysatorlösung in einem Kneter gemischt.

Zur Katalysatorpaste werden gemäß Tabelle (1) Additive zu 0,1 Gew.-% zugesetzt und homogen verknetet. Basis- und Katalysatorpaste werden in eine Doppelkammerkartusche gefüllt und die Standfestigkeit der einzelnen Abformmassen gemäß nachfolgendem Test bestimmt.

### Vergleichsbeispiel Stand der Technik

Basis- und Katalysatorpaste werden analog dem Anwendungsbeispiel hergestellt, jedoch wird kein Vertreter der erfindungsgemäßen Komponente (h) zugesetzt, sondern statt dessen die Aerosilmenge (silanisierte pyrogene Kieselsäure) um 2 % erhöht. Die Standfestigkeit wird analog dem folgenden Test durchgeführt.

### Standfestigkeitsuntersuchung

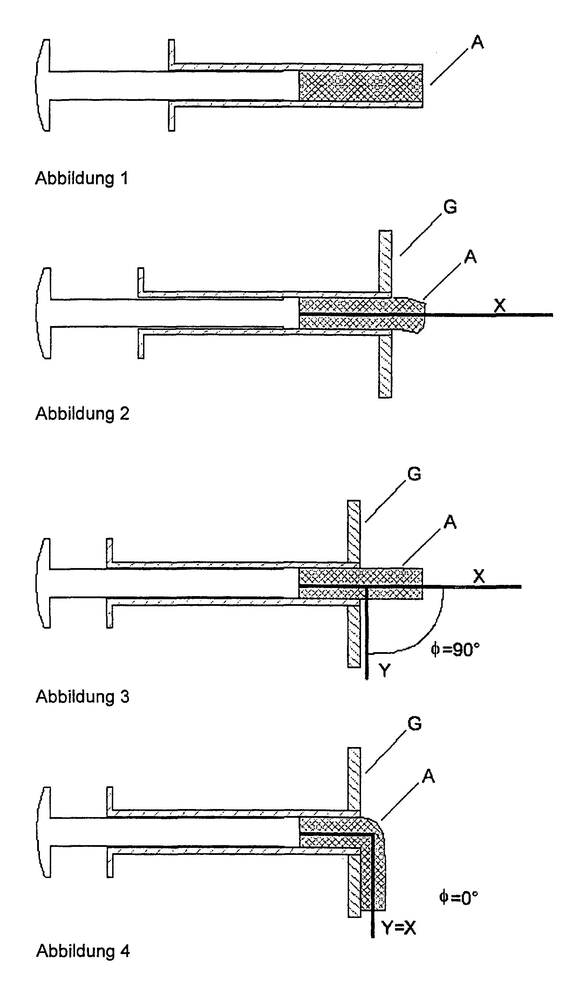

Eine an der Spitze abgesägte Elastomerspritze (Abbildung 1) wird mit angemischter Abformmasse (A) befüllt. Das offene Ende der Spritze wird in die Bohrung einer Glasplatte (G) eingesetzt und durch Druck auf den Kolben die Abformmasse (A) aus der Spritze über den Rand der Glasplatte (G) in Richtung (X) hinaus befördert (Abbildung 2). Die Standfestigkeit der Abformmasse wirkt der Schwerkraft entgegen. Je standfester die Masse ist, desto näher ist der Winkel φ zwischen einer Parallelen (Y) zur Glasplatte (G) und einer idealisierten Geraden (X) durch den ausgebrachten Strang Abformmasse (A) an 90° (Abbildung 3). Wenig oder nicht standfeste Proben gleiten an der Glaswand (G) ab und bewirken einen Winkel φ von 0° (Abbildung 4).

### Untersuchung der Ausdrückkraft

In einer Universalprüfmaschine (Fa. Zwick) wird eine handelsübliche Doppelkammerkartusche eingespannt, die mit den jeweiligen Basis- und Katalysatorpasten befüllt ist. Es wird kein statischer Mischer aufgesetzt. Ein Verschluß der Doppelkammerkartuschen erfolgt wie bei handelsüblichen Produkten auf der Basis- und Katalysatorseite jeweils durch einen Plastikstopfen mit einem Gummiring als Dichtlippe. Die Pasten werden in der Prüfmaschine aus den Kartuschen ausgedrückt, indem ein Stempel auf die Plastikstopfen Basis- und Katalysator-seitig drückt. Als Vorschubgeschwindigkeit wurden 50 mm/min und als Vorkraft 10 N eingestellt.

**Tabelle (1):**

| Standfestigkeiten und Ausdrückkräfte | | | | |
|---|---|---|---|---|
| **Additiv** | **Additiv- Variante** | **Stand- festigkeit** | **Ausdrück- kraft** | **Kontakt- winkel** |
| Keines, Vergleichsbeispiel | | 0° | 578 N | > 80° |
| Herstellungsbeispiel Additiv | Inert | 90° | 531 N | > 80° |
| Synperonic PE/L 121 (Hersteller ICI) | Nicht inert | 90° | 570 N | > 80° |
| Diol 6000 | Nicht inert | 90° | 543 N | > 80° |
| Polypropylenglycol M_{w}≈4000 | Nicht inert | 90° | 558 N | > 80° |
| Keines, Vergleichsbeispiel Stand der Technik | | 90° | 800 N | > 80° |

Der Zusatz von erfindungsgemäßer Komponente (h) führt bei nicht-signifikanter Veränderung der Ausdruckkräfte zur Erhöhung der Standfestigkeit.

## Patentansprüche

1. Additionsvemetzendes Abformmaterial auf Silikonbasis, enthaltend
(a) Organopolysiloxane mit mindestens zwei ungesättigten Gruppen im Molekül,
(b) Organohydrogenpolysiloxane mit mindestens drei Si-H-Gruppen im Molekül,
(c) gegebenenfalls Organopolysiloxane ohne reaktive Gruppen,
(d) Platinkatalysator,
(e) gegebenenfalls Hydrophilierungsmittel,
(f) Füllstoff,
(g) gegebenenfalls weitere übliche Zusatz-, Hilfs- und Farbstoffe,
**dadurch gekennzeichnet, daß** es als Komponente (h) Polyalkylenoxid und/oder dessen Derivate mit einer mittleren Molmasse M_{w} > 3000 zu 0,001 bis 1,0 Gew.-%, bezogen auf die Gesamtmasse des ausgehärteten Materials, enthält.

2. Abformmaterial nach Anspruch 1, enthaltend
10 bis 40 Gew.-% Komponenten (a) + (b)
0 bis 40 Gew.-% Komponente (c)
0,00005 bis 0,05 Gew.-% Komponente (d)
0 bis 10 Gew.-% Komponente (e)
10 bis 79,99895 Gew.-% Komponente (f)
0 bis 2 Gew.-% Komponente (g)
0,001 bis 1,0 Gew.-% Komponente (h)
jeweils bezogen auf das Gesamtgewicht der Masse.

3. Abformmaterial nach einem der Ansprüche 1 oder 2, **dadurch gekennzeichnet, daß** die Komponente (h) ein Polyalkylenoxid- oder -derivat der allgemeinen Formel (2) darstellt:
R-X-[(CH₂)ₙ-(CHR')-O]ₖ-[(CH₂)ₘ-(CHR")-O]ₗ-(CH₂)ₘ-(CHR")-X-R (2)
worin bedeuten:
n = 1 bis 6, wobei n innerhalb der Kette variieren kann,
m = 1 bis 6, wobei m innerhalb der Kette variieren kann,
k, l = 2 bis 500,
R', R" = H, Methyl, Ethyl,
X = S, O, NH,
R = H oder C₁₋₁₈-Alkyl, oder Carbonyl-C₁₋₁₇-Alkyl, oder ein Rest der allgemeinen Formel (3):
-CO-Xₚ-R''' (3)
worin R''' = C₁₋₁₈-Alkyl und/oder C₆₋₁₈-Aryl bedeutet und X die oben angegebene Bedeutung hat und p = 0 oder 1 ist,
wobei die mit den Symbolen k und l indizierten Klammerausdrücke regelmäßig oder unregelmäßig alternierend oder in Blockform angeordnet sein können.

4. Abformmaterial nach Anspruch 3, **dadurch gekennzeichnet, daß** das Polyalkylenoxidderivat der Formel: 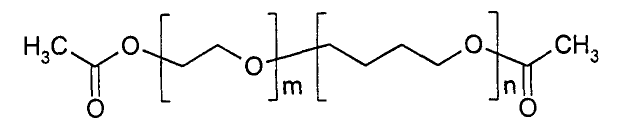 oder der Formel 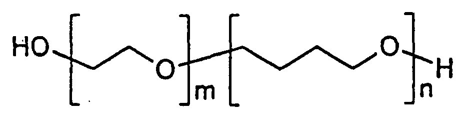 entspricht, worin das Verhältnis von m : n = 1 : 3 bis 1 : 4 beträgt.

5. Abformmaterial nach einem der Ansprüche 1 oder 2, **dadurch gekennzeichnet, daß** das Polyalkylenoxid oder -derivat ein Blockcopolymeres des Propylenoxids und Ethylenoxids darstellt.

6. Abformmaterial nach einem der Ansprüche 1 bis 5, **dadurch gekennzeichnet, daß** es in Form einer Basispaste und einer davon räumlich getrennten Katalysatorpaste vorliegt, wobei die gesamte Komponente (b) in der Basispaste und die gesamte Komponente (d) in der Katalysatorpaste vorhanden sind und die übrigen Komponenten wahlweise in den beiden Pasten verteilt sind.

7. Verwendung des Abformmaterials nach einem der Ansprüche 1 bis 6 im zahnmedizinischen Bereich, insbesondere als Bißregistrat.

8. Verwendung von Polyalkylenoxiden und/oder deren Derivaten mit einer mittleren Molmasse M_{w} > 3000 als Zusatz zu Abformmassen auf Silikonbasis zur Erhöhung des Standvermögens.

## Claims

1. Silicone-based addition-crosslinking impression material, containing
(a) organopolysiloxanes with at least two unsaturated groups in the molecule,
(b) organohydrogen polysiloxanes with at least three Si-H groups in the molecule,
(c) optionally, organopolysiloxanes without reactive groups,
(d) platinum catalyst,
(e) optionally, hydrophilizing agent,
(f) filler,
(g) optionally, further customary additives, auxiliaries and dyes,
**characterized in that** the impression material contains, as component (h), polyalkylene oxide and/or its derivatives with an average molar mass M_{w} > 3000 at the rate of 0.001 to 1.0 wt.-% relative to the total mass of the cured material.

2. Impression material according to Claim 1, containing
10 to 40 wt.-% components (a)+(b)
0 to 40 wt.-% component (c)
0.00005 to 0.05 wt.-% component (d)
0 to 10 wt.-% component (e)
10 to 79.99895 wt.-% component (f)
0 to 2 wt.-% component (g)
0.001 to 1.0 wt.-% component (h)
each relative to the total weight of the composition.

3. Impression material according to one of Claim 1 or 2, **characterized in that** component (h) represents a polyalkylene oxide or derivative of the general formula (2):
R-X-[(CH₂)ₙ-(CHR')-O]ₖ-[(CH₂)ₘ-(CHR")-O]ₗ-(CH₂)ₘ-(CHR")-X-R (2)
in which:
n = 1 to 6, n being able to vary within the chain,
m = 1 to 6, m being able to vary within the chain,
k, l = 2 to 500,
R', R" = H, methyl, ethyl,
X = S, O, NH,
R = H or C₁₋₁₈ alkyl, or carbonyl C₁₋₁₇ alkyl, or a radical of the general formula (3):
-CO-Xₚ-R''' (3)
where R'" = C₁₋₁₈ alkyl and/or C₆₋₁₈ aryl and X has the meaning given above and p = 0 or 1,
the bracketed expressions subscripted with the symbols k and l being able to be arranged regularly or irregularly alternating or in block form.

4. Impression material according to Claim 3, **characterized in that** the polyalkylene oxide derivative corresponds to the formula: 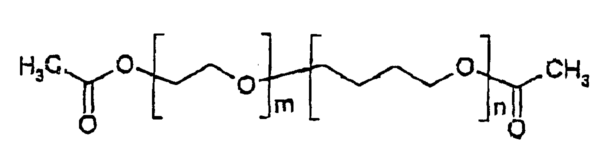 or the formula 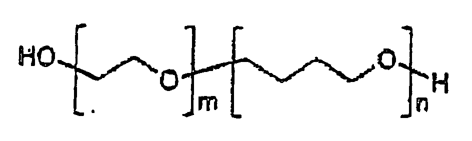 where the ratio of m:n = 1:3 to 1:4.

5. Impression material according to one of Claim 1 or 2, **characterized in that** the polyalkylene oxide or derivative represents a block copolymer of propylene oxide and ethylene oxide.

6. Impression material according to one of Claims 1 to 5, **characterized in that** it is present in the form of a base paste and a catalyst paste physically separated from it, the total component (b) being present in the base paste and the total component (d) being present in the catalyst paste and the remaining components being distributed as desired in the two pastes.

7. Use of the impression material according to one of Claims 1 to 6 in dentistry, in particular as a bite registration.

8. Use of polyalkylene oxides and/or their derivatives with an average molar mass M_{w} > 3000 as additives to the silicone-based impression compositions to improve the dimensional stability.

## Revendications

1. Matériau de moulage réticulant par addition à base de silicone, contenant
(a) des organopolysiloxanes présentant au moins deux groupes insaturés par molécule,
(b) des organohydrogénopolysiloxanes présentant au moins trois groupes Si-H par molécule,
(c) le cas échéant des organopolysiloxanes sans groupes réactifs,
(d) un catalyseur de platine,
(e) le cas échéant un agent hydrophile,
(f) des charges,
(g) le cas échéant d'autres additifs, adjuvants et colorants usuels,
**caractérisé en ce qu'**il contient, comme composant (h), du poly(oxyde d'alkylène) et/ou ses dérivés présentant une masse molaire moyenne M_{w}>3000 à raison de 0,001 à 1,0% en poids par rapport à la masse totale du matériau durci.

2. Matériau de moulage selon la revendication 1, contenant
10 à 40% en poids de composants (a) + (b)
0 à 40% en poids de composant (c)
0,00005 à 0,05% en poids de composant (d)
0 à 10% en poids de composant (e)
10 à 79,99895% en poids de composant (f)
0 à 2% en poids de composant (g)
0,001 à 1,0% en poids de composant (h)
à chaque fois par rapport au poids total de la masse.

3. Matériau de moulage selon l'une quelconque des revendications 1 ou 2, **caractérisé en ce que** le composant (h) est un poly(oxyde d'alkylène) ou un dérivé de celui-ci de formule générale (2)
R-X-[(CH₂)ₙ-(CHR')-O]ₖ-[(CH₂)ₘ-(CHR")-O]ₗ-(CH₂)ₘ-(CHR")-X-R (2)
dans laquelle :
n = 1 à 6, n pouvant varier dans la chaîne,
m = 1 à 6, m pouvant varier dans la chaîne,
k, l = 2 à 500,
R', R" = H, méthyle, éthyle,
X = S, O, NH,
R = H ou alkyle en C₁ à C₁₈, ou carbonylalkyle en C₁ à C₁₇, ou un radical de formule générale (3) :
-CO-Xₚ-R''' (3)
dans laquelle R' '' = de préférence alkyle en C₁ à C₁₈ et/ou aryle en C₆ à C₁₈, X a la signification susmentionnée et p = 0 ou 1,
les parties entre parenthèses, présentant les indices k et l, pouvant être disposées de manière régulière ou irrégulière, alternativement ou sous forme de blocs.

4. Matériau de moulage selon la revendication 3, **caractérisé en ce que** le dérivé de poly(oxyde d'alkylène correspond à la formule 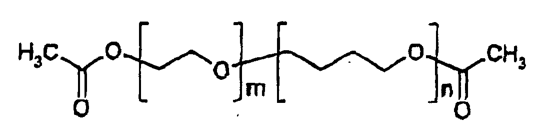 ou à la formule 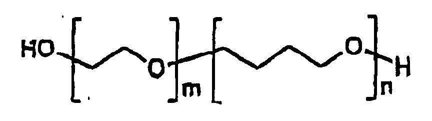 le rapport m : n étant de 1 : 3 à 1 : 4.

5. Matériau de moulage selon l'une quelconque des revendications 1 ou 2, **caractérisé en ce que** le poly(oxyde d'alkylène) ou son dérivé est un copolymère à blocs d'oxyde de propylène et d'oxyde d'éthylène.

6. Matériau de moulage selon l'une quelconque des revendications 1 à 5, **caractérisé en ce qu'**il se trouve sous forme d'une pâte de base et d'une pâte de catalyseur séparée dans l'espace de celle-ci, la totalité du composant (b) se trouvant dans la pâte de base et la totalité du composant (d) dans la pâte de catalyseur et les autres composants étant répartis au choix dans les deux pâtes.

7. Utilisation du matériau de moulage selon la revendication 1 à 6 dans le domaine de la médecine dentaire, en particulier pour la prise d'empreintes occlusives.

8. Utilisation de poly(oxydes d'alkylène) et/ou de leurs dérivés présentant une masse molaire moyenne M_{w}>3000 comme additif dans des masses de moulage à base de silicone pour augmenter la résistance au fluage.
